# EUROPEAN PATENT SPECIFICATION

(11) **EP 4 624 906 B1**
(45) Date of publication and mention of the grant of the patent: **11.11.2026**
(21) Application number: 25157717.7
(22) Date of filing: 13.02.2025
(51) Int. Cl.: G01N 21/84, G01N 21/27, G01N 23/22, G01N 21/3563, G01N 21/3581, G01N 21/359, G01N 21/65

(54) **NONDESTRUCTIVE DETERMINATION OF COATING MATERIAL COMPOSITION**
ZERSTÖRUNGSFREIE BESTIMMUNG EINER BESCHICHTUNGSMATERIALZUSAMMENSETZUNG
DÉTERMINATION NON DESTRUCTIVE D'UNE COMPOSITION DE MATÉRIAU DE REVÊTEMENT

(30) Priority: 29.03.2024 US 202418622598
(43) Date of publication of application: 01.10.2025
(73) Proprietor: The Boeing Company, Arlington, VA 22202 (US)
(72) Inventor: BRENT-FULPS, Emily L., Arlington, 22202 (US); VAHEY, Paul Griffin, Arlington, 22202 (US); SCHULTZ, Karen A., Arlington, 22202 (US); FIFE, Scott, Arlington, 22202 (US); CHEN, Alina, Arlington, 22202 (US); COLLIGAN, Jonathan Thomas, Arlington, 22202 (US); HOUSE, Geoffrey Michael, Arlington, 22202 (US); PARKER, George Alexander, Arlington, 22202 (US); STRAUS, Lori Clarice, Arlington, 22202 (US)
(74) Representative: Boult Wade Tennant LLP

(56) References cited:
- EP-A1- 4 368 974
- EP-B1- 2 108 939
- CN-B- 108 152 102
- US-A1- 2008 032 037
- US-A1- 2013 342 824
- VIGERUST B ET AL: "QUANTITATIVE ANALYSIS OF ADDITIVES IN LOW-DENSITY POLYETHYLENE USING INFRARED SPECTROSCOPY AND MULTIVARIATE CALIBRATION", APPLIED SPECTROSCOPY, THE SOCIETY FOR APPLIED SPECTROSCOPY. BALTIMORE, US, vol. 45, no. 2, 1 February 1991 (1991-02-01), pages 173 - 177, XP000175854, ISSN: 0003-7028, DOI: 10.1366/0003702914337605

## Description

### BACKGROUND

Paint and primer compositions can be used to form coatings on surfaces. Paint and primer serve complementary roles. Primer generally refers to a composition that forms a preparatory coating applied to surfaces before the application of paint. The primer can provide a smooth and stable base for the paint to adhere to. Paint generally refers to a composition that forms a top coating over the primer, providing color, protection, and aesthetic appeal to the surface.

A paint or primer composition generally includes a pigment and/or a dye, which are fine particles that impart color, opacity, and/or other properties to the composition. The pigment and/or the dye is dispersed in a matrix, also known as a binder. The binder holds the composition together and adheres the composition to a surface of a substrate. Some examples of binder materials include acrylics, polyurethanes, and epoxies. Specific formulations of the composition can vary between different types of paints and primers, as well as based on intended applications and properties of the composition.

US 2013/342824 A1, in accordance with its abstract, states a system and method for providing a protective coating for a substrate that may be inspected using the unaided eye or other apparatus under available light. The protective coating is mixed with an additive comprising flakes or particles that, when applied to the substrate as part of the protective coating, allow the user to empirically determine if the surface has received an adequate protective coat. The determination of whether or not any defects exist may include comparing the observed appearance of the specialty pigment particles with a comparative standard.

US 2008/032037 A1, in accordance with its abstract, states a method of determining conditions at least necessary for radiation curing pigmented radiation-curable coating materials, and an associated apparatus.

EP 2 108 939 B1, in accordance with its abstract, states a method that involves capturing a space-resolved transmission image of an expandable polystyrene component, an area of the component, or volume that is completely translucent or translucent in a region of electromagnetic radiation, by two-dimensional radiation of the component, the area or the volume using laser beam. An influence of scattered radiation on the image is reduced by spatial filtering. The image is corrected using geometric data of the component, the area, or the volume, in order to obtain a thickness profile of the component, the area, or the volume based on the image. A method for determining moisture content of a component is also stated.

Vigerust B et al: "Quantitative Analysis of Additives in Low-Density Polyethylene Using Infrared Spectroscopy and Multivariate Calibration", Applied Spectroscopy, The Society For Applied Spectroscopy, Baltimore, US, vol. 45, no. 2 (1991-02-01), pages 173-177, states a method for determining the concentration of silica, erucamide and butylhydroxytoluene (BJT) based on infrared (IR) spectroscopy and a calibration model.

CN 108 152 102 B, in accordance with its abstract, states a method for quantitative analysis of a filler in a coating that comprises at least the following steps: a. taking the coating, and adding an organic solvent thereto for extraction to obtain an insoluble matter A; b. adding tetrahydrofuran to the insoluble matter A, ultrasonically dispersing, and separating an insoluble solid B therein by centrifugation, the volume ratio of insoluble matter A to tetrahydrofuran being 1 to (1 to 3); c. putting the insoluble solid B in a vacuum drying oven, and drying under vacuum at a temperature of 50 DEG C for 12 to 24 hours to obtain a mixed filler; d. grinding the mixed filler into a powder for qualitative and quantitative analysis.

EP 4 368 974 A1, in accordance with its abstract, states a device for inspecting containers (10), comprising a transport device, which transports the containers along a predefined transport path, an inspection device, which inspects a base region of the containers, characterized in that a coating of the base region of the containers can be inspected by the inspection device. This document is comprised in the state of the art pursuant to Article 54(3) EPC.

### SUMMARY

There is a method provided for determining a composition of a coating material on a sample. The method comprises obtaining calibration data that correlates a ratio of matrix material to additive material with results of nondestructive spectral analysis; performing nondestructive spectral analysis on the sample; and comparing results of the nondestructive spectral analysis on the sample to the calibration data to determine a ratio of matrix material to additive material in the sample. The step of obtaining the calibration data that correlates the ratio of matrix material to additive material with results of nondestructive spectral analysis comprises: creating a plurality of reference standards by applying a reference material to a reference substrate, wherein the reference material comprises a different ratio of a matrix material to an additive material for each of the plurality of reference standards; performing nondestructive spectral analysis on each of the plurality of reference standards; and subjecting each of the plurality of reference standards to destructive analysis to characterize the ratio of the matrix material to the additive material in each of the plurality of reference standards, and to correlate results of the destructive analysis and the nondestructive spectral analysis. Subjecting each of the plurality of reference standards to the destructive analysis comprises performing thermogravimetric analysis. The coating material is a paint and/or primer.

This simplified summary of the specification is presented to provide a basic understanding of some features of the specification. This summary is not an extensive overview of the specification. It is intended to neither identify key or critical elements of the specification nor delineate any particular examples of the specification, or any scope of the claims. Its sole purpose is to present some concepts of the specification in a simplified form as a prelude to the more detailed description that is presented in this disclosure.

### BRIEF DESCRIPTION OF THE DRAWINGS

FIG. 1 shows an example of an aircraft comprising one or more components with surfaces that can be coated with a coating material.
FIG. 2 shows an exploded view of a surface, primer, and paint of a component of FIG. 1.
FIG. 3 shows an example of a system for determining a composition of a coating material on a surface, such one of the one or more surfaces of FIG. 1.
FIGS. 4A-4B show a block diagram of an example method for determining a composition of a coating material.
FIG. 5 is a block diagram of an example computing device.

### DETAILED DESCRIPTION

As introduced above, a paint or primer composition generally includes a pigment and/or a dye dispersed in a matrix. Specific formulations of the composition can vary between different types of paints and primers, as well as based on intended applications and properties of the composition.

However, improper mixing and/or decanting can affect the properties and overall quality of the painted surface. Uneven distribution of pigments, fillers, or dyes within the matrix can result in variations in color on the painted surface that deviate from specifications for the painted surface. Insufficient mixing can additionally or alternatively result in uneven coverage. Some areas may have too much pigment, causing a thick and opaque appearance, while other areas may have too little, leading to a translucent or patchy finish. Another potential issue is that improperly mixed paint may not adhere well to the surface, leading to issues such as peeling, cracking, or flaking. This can compromise the durability and longevity of the finish and render the underlying surface susceptible to corrosion, fluid ingress, weathering, mechanical wear, cracking, etc. Inconsistent mixing can additionally or alternatively create texture problems, such as grittiness or a sandy feel on the painted surface. This can affect the smoothness and overall quality of the finish. Paint that is not thoroughly mixed may additionally or alternatively have difficulties drying or curing properly. This can result in extended drying times, an uneven finish, or a tacky surface that attracts dirt and debris.

In some instances, thermogravimetric analysis (TGA) can be used to assess the composition of a paint or primer mixture before it is applied to a surface. This can result in a reliable determination of whether the paint or primer is properly mixed before it is applied to the surface. However, TGA is a destructive analytical method that results in loss of the sample. As a result, if TGA is used to analyze paint or primer after application to a product, repair and re-application would be required.

Accordingly, examples are disclosed that relate to a non-destructive test method for cured coatings (paints and/or primers) to determine the composition of the applied coatings. The method comprises creating a plurality of reference standards by applying a reference material to a reference substrate. The reference material comprises a different ratio of a matrix material to an additive material for each of the plurality of reference standards. The method further comprises performing nondestructive spectral analysis on each of the plurality of reference standards. The method further comprises subjecting each of the plurality of reference standards to destructive analysis to characterize the ratio of the matrix material to the additive material in each of the plurality of reference standards and to correlate results of the destructive analysis and the nondestructive spectral analysis.

The nondestructive spectral analysis is performed on the sample. The method further comprises comparing results of the nondestructive spectral analysis on the sample to the correlated results of the destructive analysis and the nondestructive spectral analysis on the plurality of the reference standards to determine the ratio of the matrix material to the additive material in the sample. In this manner, the composition (e.g., ratios of ingredients) of the coating can be determined as it is on the part, rather than as supplied (e.g., in a container such as a bucket).

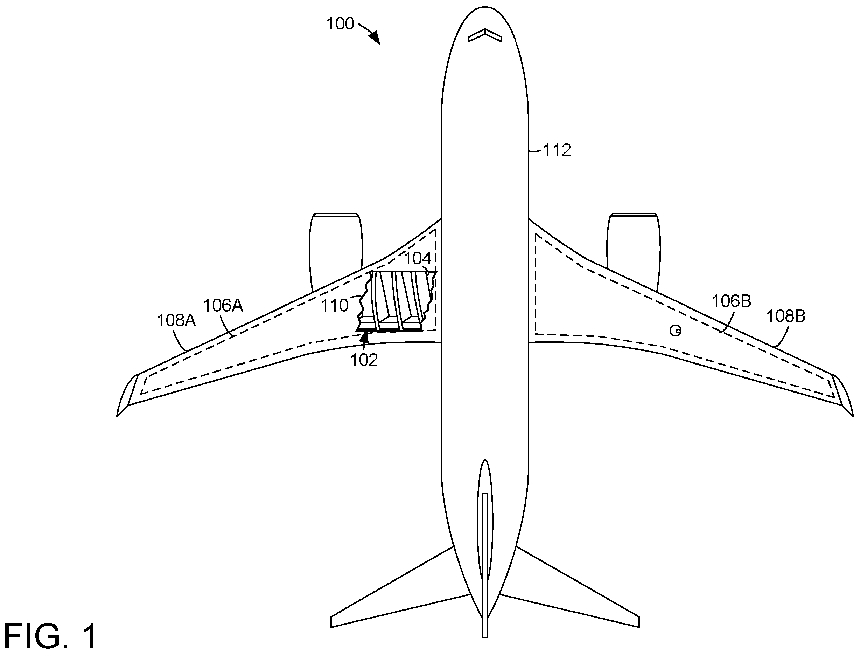

Prior to discussing this methodology in detail, FIG. 1 illustrates examples of surfaces on an aircraft 100 that can be coated with one or more layers of a paint or a primer. In some examples, the paint and/or primer is applied to a spar 102 or a rib 104 within a fuel tank 106A or 106B located on a wing 108A or 108B of the aircraft. In other examples, the paint and/or the primer can be applied to any other suitable surface. Other examples of suitable surfaces include a skin 110 of the fuel tank 106A, 106B, and a fuselage 112 of the aircraft 100.

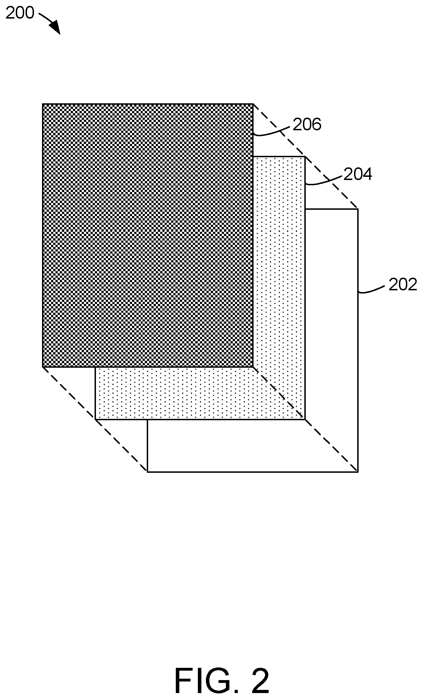

FIG. 2 shows an exploded view of a surface 200. In some examples, the surface 200 comprises an internal or external surface of an aircraft component, such as the fuel tank 106A or 106B. The surface 200 comprises a substrate 202. A bottom coating of primer 204 is disposed on the substrate 202. A top coating of paint 206 is disposed on the primer 204. In some examples, the paint 206 and the primer 204 protect the substrate 202 from corrosion. Exposure to environmental factors (e.g., moisture, salt, fuel) can lead to corrosion. The paint 206 and the primer 204 act as a protective barrier, preventing direct contact between the substrate 202 and environmental factors. The paint 206 and the primer 204 can additionally or alternatively act as a protective sealant, covering seams, joints, and fasteners. This helps prevent water and other contaminants from infiltrating the aircraft structure, reducing the risk of internal corrosion, and preventing fuel and other substances from leaking.

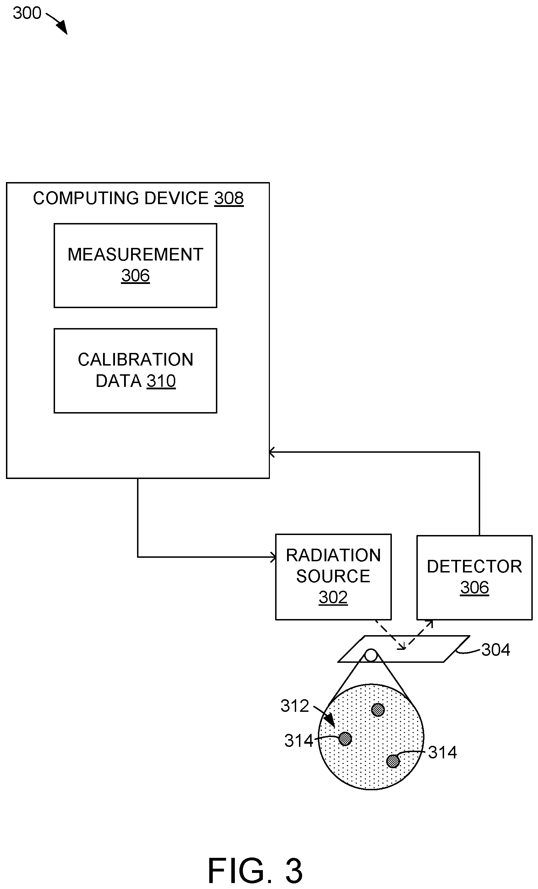

With reference now to FIG. 3, an example of a system 300 is illustrated for determining a composition of a coating material, such as the paint 206 and/or the primer 204 of FIG. 2, on a sample. The system 300 comprises a radiation source 302. In some examples, the radiation source 302 comprises an electromagnetic radiation source. The electromagnetic radiation source is configured to emit electromagnetic radiation at a predetermined wavelength or range of wavelengths (e.g., x-rays, ultraviolet light, visible light, or infrared light). In other examples, the radiation source 302 can emit particles, such as electrons.

The radiation source 302 is configured to irradiate a sample 304, such as the surface 200 of FIG. 2. The system 300 also includes a detector 306 configured to measure radiation reflected or emitted from the sample 304. The detector 306 is further configured to output a measurement of the radiation reflected or emitted from the sample 304 to a computing device 308. In this manner, the computing device 308 can use the detector 306 to spectroscopically analyze the sample 304.

Spectroscopic analysis of the sample 304 is based on calibration data 310 determined based upon a plurality of reference standards. The plurality of reference standards are determined by applying a reference material to a reference substrate. Like the sample 304, the reference material comprises a mixture of a matrix material 312. In some examples, the matrix material 312 comprises a cured polymeric resin. Some suitable examples of polymeric resins include an epoxy resin, a polyurethane resin, and an acrylic resin. It will also be appreciated that any other suitable matrix can be used. Other examples of suitable matrix materials include alkyd resins, polyester resins, vinyl resins, silicone resins, and fluoropolymer resins.

The reference material further comprises an additive material 314. In some examples, the additive material 314 comprises a pigment. It will also be appreciated that any other suitable additive material can be used. Another example of a suitable additive material is a dye. The additive material alters one or more properties of the reference material, such as its color and chemical resistance.

Each reference standard comprises a different ratio of the matrix material 312 to the additive material 314. The composition of the reference standard can be defined based on an expected ratio of the additive material to the matrix material in an experimental sample, and the densities of the additive material and the matrix material. In some examples, the additive material 314 comprises 0 - 80 wt.% of the sample 304. In some, more specific examples, the additive material 314 comprises 0-70 wt.% of the sample 304. In further, more specific examples, the additive material 314 comprises 0-50 wt.% of the sample 304. Covering a range of anticipated concentrations ensures accurate calibration.

A representative number of reference standards are prepared that reflect anticipated diversity of samples encountered in experimental analysis. The number of representative samples also considers statistical factors, such as precision and confidence level. Increasing the number of samples results in greater statistical reliability and provides a more robust calibration than using fewer samples. Replicate reference standards can also help to evaluate the precision or repeatability of the calibration. In some examples, the number of reference standards is in a range of 2-100. In some, more specific examples, the number is in a range of 3-27. In further, more specific examples, the number is in a range of 5-12.

Nondestructive spectral analysis is performed on each of the plurality of reference standards. In some examples, performing the nondestructive spectral analysis comprises performing one or more of x-ray fluorescence (XRF) spectroscopy, vibrational spectroscopy (e.g., infrared (IR) spectroscopy, near-IR (NIR) spectroscopy, terahertz (THz) spectroscopy, or Raman spectroscopy), x-ray photoelectron (XPS) spectroscopy, or energy-dispersive x-ray (EDX) spectroscopy.

It will also be appreciated that two or more types of nondestructive spectral analysis can be performed on each of the plurality of reference standards. Using two or more analytical methods together can offer several advantages. For example, combining different analytical methods can provide a more complete picture of the sample's characteristics. Utilizing methods with different sensitivities and selectivities can additionally or alternatively increase the overall accuracy of the analysis, and/or cover a broader range of sample characteristics.

Each of the plurality of reference standards is further subjected to destructive analysis. The destructive analysis comprises TGA. In some examples, the destructive analysis further comprises any other suitable analysis. Other examples of suitable destructive analysis methods include destructive chromatographic analysis, chemical reaction of the sample (e.g., titration), and solution-phase nuclear magnetic resonance (NMR) spectroscopy. As introduced above, the destructive analysis can characterize the ratio of the matrix material to the additive material in each of the plurality of reference standards. In this manner, a known ratio can be correlated to the nondestructive spectral analysis of each reference standard.

In some examples, results of one or more additional testing methods can be correlated to the results of the nondestructive spectral analysis. For example, the results of the nondestructive spectral analysis can be further correlated to one or more of durability, adhesion, corrosion resistance, color retention, gloss retention, chemical resistance, ease of application, flexibility, elasticity, or abrasion resistance. In this manner, the nondestructive spectral analysis can also be used to gauge one or more additional properties of the sample and infer performance of the cured coating.

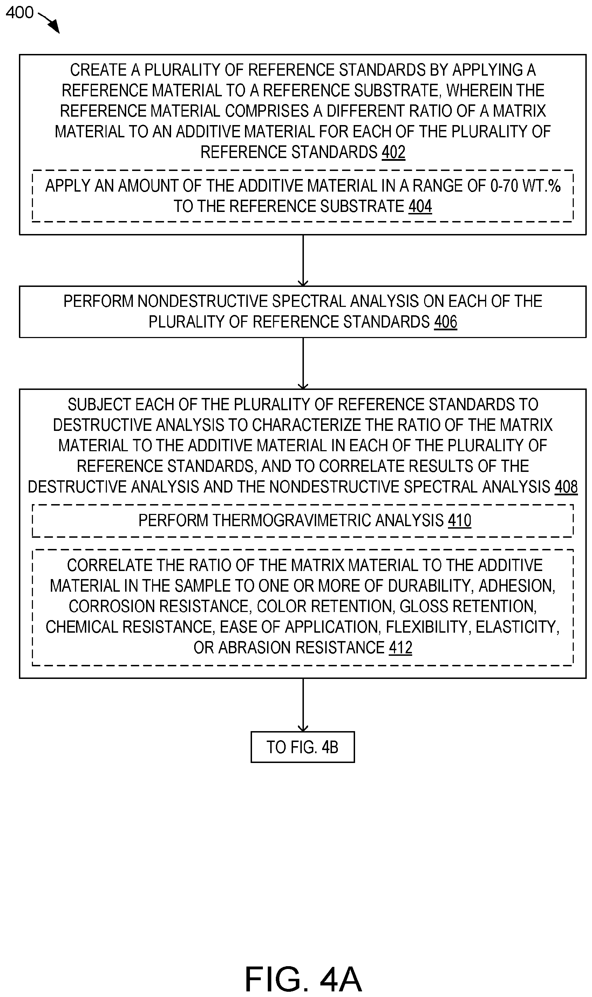

FIGS. 4A-4B show a flow diagram depicting an example method 400 for determining a composition of a coating material on a sample. The following description of the method 400 is provided with reference to the components described above and shown in FIGS. 1-3 and 5. It will be appreciated that the method 400 also can be performed in other contexts.

Referring first to FIG. 4A, at 402, the method 400 comprises creating a plurality of reference standards by applying a reference material to a reference substrate, wherein the reference material comprises a different ratio of a matrix material to an additive material for each of the plurality of reference standards. In some examples, the plurality of reference standards can be created by applying paint and/or primer to the reference substrate to create a coated surface such as the surface 200 of FIG. 2. The reference standards are used to calibrate the method 400 by correlating results of a nondestructive spectral analysis method with results of destructive analysis. In this manner, the nondestructive spectral analysis can be used independently of the destructive analysis to inspect a coating *in situ* without removing the coating from the coated surface.

In some examples, at 404, applying the reference material to the reference substrate comprises applying an amount of the additive material in a range of 0-70 wt.% to the reference substrate. As described above, the composition and number of reference standards can be determined based upon an expected ratio of the additive material to the matrix material in an experimental sample to enable accurate and precise measurement of the ratio of the matrix material to the additive material.

At 406, the method 400 includes performing nondestructive spectral analysis on each of the plurality of reference standards. As introduced above, the nondestructive spectral analysis can include one or more nondestructive spectral analysis techniques. Some examples of suitable nondestructive spectral analysis techniques include XRF, IR, NIR, THz, Raman, XPS, or EDX spectroscopy.

The method 400 further comprises, at 408, subjecting each of the plurality of reference standards to destructive analysis. In some examples, at 410, subjecting each of the plurality of reference standards to the destructive analysis comprises performing TGA. In other examples, the destructive analysis additionally or alternatively includes any other suitable destructive analysis techniques. Other examples of destructive analysis techniques include solution-phase chromatography and NMR spectroscopy. As described above, the destructive analysis is used to characterize the ratio of the matrix material to the additive material in each of the plurality of reference standards. This results in a known ratio that can be used to correlate results of the destructive analysis and the nondestructive spectral analysis.

At 412, in some examples, the method 400 further comprises correlating the ratio of the matrix material to the additive material in the sample to one or more of durability, adhesion, corrosion resistance, color retention, gloss retention, chemical resistance, ease of application, flexibility, elasticity, or abrasion resistance. As described above, this can be accomplished by correlating results of one or more additional testing methods to the results of the nondestructive spectral analysis. In this manner, the nondestructive spectral analysis can also be used to infer other properties of the cured coating.

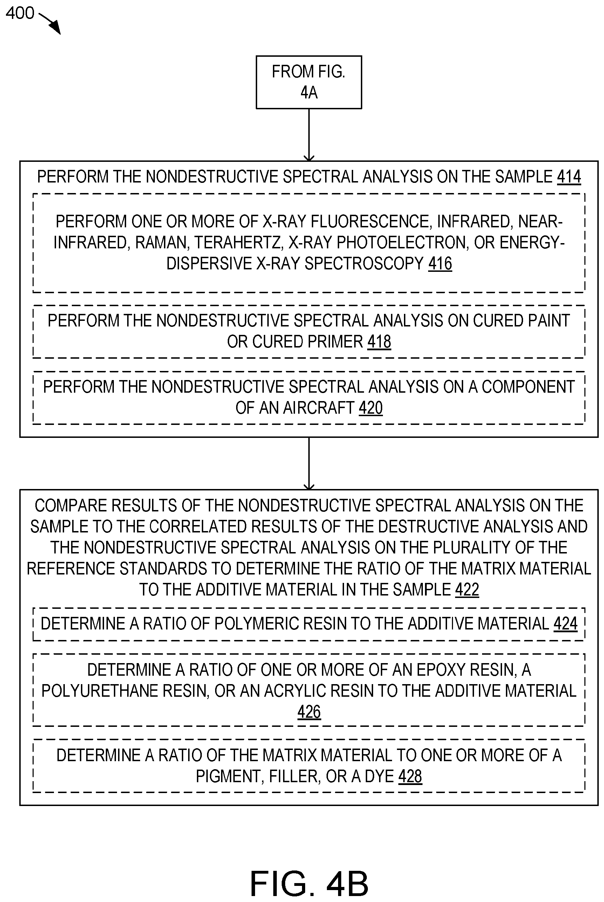

Referring now to FIG. 4B, at 414, the method 400 includes performing the nondestructive spectral analysis on the sample. In some examples, at 416, performing the nondestructive spectral analysis comprises performing one or more of XRF, IR, NIR, Raman, THz, XPS, or EDX spectroscopy.

In some examples, the nondestructive spectral analysis can be performed on the same equipment as the nondestructive spectral analysis of step 406. For example, the spectral analysis of steps 406 and 414 can be performed using the detector 306 of FIG. 3. It will also be appreciated that the nondestructive spectral analysis of step 406 and the nondestructive spectral analysis of step 414 can be performed using different equipment. In this manner, the nondestructive spectral analysis can be calibrated at a first location using a first spectroscopy system (e.g., in a research or quality assurance laboratory), and the results of the calibration can be used to perform the nondestructive spectral analysis at a second, different location and/or using a second, different spectroscopy system (e.g., on a factory production line with a portable measurement device).

At 418, in some examples, performing the nondestructive spectral analysis on the sample comprises performing the nondestructive spectral analysis on cured paint or a cured primer. For example, the nondestructive spectral analysis can be used to determine the composition of the primer 204 and/or the paint 206 of the surface 200 of FIG. 2.

In some examples, at 420, performing the nondestructive spectral analysis on the sample comprises performing the nondestructive spectral analysis on a component of an aircraft. For example, the nondestructive spectral analysis can be performed on a spar 102 or a rib 104 of the aircraft 100 of FIG. 1.

At 422, the method 400 includes comparing results of the nondestructive spectral analysis on the sample to the correlated results of the destructive analysis and the nondestructive spectral analysis on the plurality of the reference standards to determine the ratio of the matrix material to the additive material in the sample. As introduced above, the results of the nondestructive spectral analysis can be interpreted on the same or different equipment than the nondestructive spectral analysis of step 406. For example, the nondestructive spectral analysis can be interpreted on the computing device 308 of FIG. 3. The computing device 308 can be the same computing device that is used to obtain the calibration data 310. In other examples, the computing device 308 can obtain the calibration data 310 from another device.

In some examples, at 424, determining the ratio of the matrix material to the additive material in the sample comprises determining a ratio of polymeric resin to the additive material. At 426, in some examples, determining the ratio of the matrix material to the additive material in the sample comprises determining a ratio of one or more of an epoxy resin, a polyurethane resin, or an acrylic resin to the additive material. In some examples, at 428, determining the ratio of the matrix material to the additive material in the sample comprises determining a ratio of the matrix material to one or more of a pigment, filler, or a dye. In this manner, the composition of the coating can be determined without destroying the sample.

In some examples, the methods and processes described herein may be tied to a computing system of one or more computing devices. In particular, such methods and processes may be implemented as a computer-application program or service, an application-programming interface (API), a library, and/or other computer-program product.

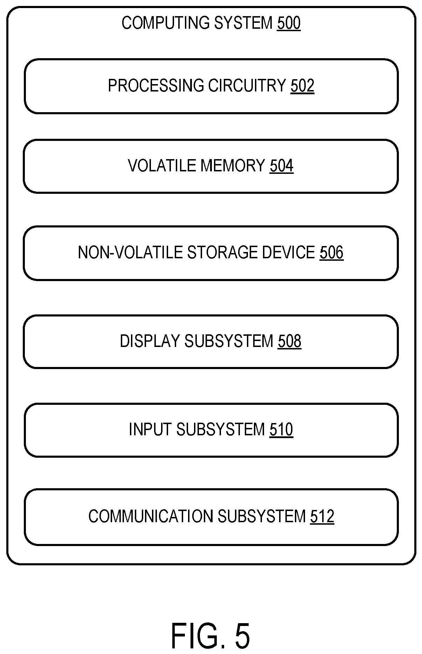

FIG. 5 schematically shows an example of a computing system 500 that can enact one or more of the methods and processes described above. Computing system 500 is shown in simplified form. Computing system 500 may embody the computing device 308 described above and illustrated in FIG. 3. Components of computing system 500 may be included in one or more personal computers, server computers, tablet computers, network computing devices, mobile computing devices, mobile communication devices (e.g., smartphone), and/or other computing devices, and wearable computing devices.

Computing system 500 includes processing circuitry 502, volatile memory 504, and a non-volatile storage device 506. Computing system 500 may optionally include a display subsystem 508, input subsystem 510, communication subsystem 512, and/or other components not shown in FIG. 5.

The processing circuitry 502 typically includes one or more logic processors, which are physical devices configured to execute instructions. For example, the logic processors may be configured to execute instructions that are part of one or more applications, programs, models, routines, libraries, objects, components, data structures, or other logical constructs. Such instructions may be implemented to perform a task, implement a data type, transform the state of one or more components, achieve a technical effect, or otherwise arrive at a desired result.

The logic processor may include one or more physical processors configured to execute software instructions. Additionally or alternatively, the logic processor may include one or more hardware logic circuits or firmware devices configured to execute hardware-implemented logic or firmware instructions. Processors of the processing circuitry 502 may be single-core or multi-core, and the instructions executed thereon may be configured for sequential, parallel, and/or distributed processing. Individual components of the processing circuitry optionally may be distributed among two or more separate devices, which may be remotely located and/or configured for coordinated processing. For example, features of the computing system disclosed herein may be virtualized and executed by remotely accessible, networked computing devices configured in a cloud-computing configuration. In such a case, these virtualized features are run on different physical logic processors of various different machines, it will be understood. These different physical logic processors of the different machines will be understood to be collectively encompassed by processing circuitry 502.

Non-volatile storage device 506 includes one or more physical devices configured to hold instructions executable by the processing circuitry to implement the methods and processes described herein. When such methods and processes are implemented, the state of non-volatile storage device 506 may be transformed-e.g., to hold different data.

Non-volatile storage device 506 may include physical devices that are removable and/or built in. Non-volatile storage device 506 may include optical memory, semiconductor memory, and/or magnetic memory, or other mass storage device technology. Non-volatile storage device 506 may include nonvolatile, dynamic, static, read/write, read-only, sequential-access, location-addressable, file-addressable, and/or content-addressable devices. It will be appreciated that non-volatile storage device 506 is configured to hold instructions even when power is cut to the non-volatile storage device 506.

Volatile memory 504 may include physical devices that include random access memory. Volatile memory 504 is typically utilized by processing circuitry 502 to temporarily store information during processing of software instructions. It will be appreciated that volatile memory 504 typically does not continue to store instructions when power is cut to the volatile memory 504.

Features of processing circuitry 502, volatile memory 504, and non-volatile storage device 506 may be integrated together into one or more hardware-logic components. Such hardware-logic components may include field-programmable gate arrays (FPGAs), program- and application-specific integrated circuits (PASIC / ASICs), program- and application-specific standard products (PSSP / ASSPs), system-on-a-chip (SOC), and complex programmable logic devices (CPLDs), for example.

The term "program" may be used to describe a feature of computing system 500 typically implemented in software by a processor to perform a particular function using portions of volatile memory, which function involves transformative processing that specially configures the processor to perform the function. Thus, a program may be instantiated via processing circuitry 502 executing instructions held by non-volatile storage device 506, using portions of volatile memory 504. It will be understood that different programs may be instantiated from the same application, service, code block, object, library, routine, API, function, etc. Likewise, the same program may be instantiated by different applications, services, code blocks, objects, routines, APIs, functions, etc. The term "program" may encompass individual or groups of executable files, data files, libraries, drivers, scripts, database records, etc.

When included, display subsystem 508 may be used to present a visual representation of data held by non-volatile storage device 506. The visual representation may take the form of a GUI. As the herein described methods and processes change the data held by the non-volatile storage device, and thus transform the state of the non-volatile storage device, the state of display subsystem 508 may likewise be transformed to visually represent changes in the underlying data. Display subsystem 508 may include one or more display devices utilizing virtually any type of technology. Such display devices may be combined with processing circuitry 502, volatile memory 504, and/or non-volatile storage device 506 in a shared enclosure, or such display devices may be peripheral display devices.

When included, input subsystem 510 may comprise or interface with one or more user-input devices such as a keyboard, mouse, touch screen, camera, or microphone.

When included, communication subsystem 512 may be configured to communicatively couple various computing devices described herein with each other, and with other devices. Communication subsystem 512 may include wired and/or wireless communication devices compatible with one or more different communication protocols. As non-limiting examples, the communication subsystem may be configured for communication via a wired or wireless local- or wide-area network, broadband cellular network, etc. In some examples, the communication subsystem may allow computing system 500 to send and/or receive messages to and/or from other devices via a network such as the Internet.

Described herein but not claimed is a computing system, comprising one or more processors configured to: receive, a measurement of electromagnetic radiation reflected or emitted from a sample, the measurement having been acquired by a nondestructive spectral analysis tool; compare the measurement of the electromagnetic radiation reflected or emitted from the sample to calibration data that correlates a ratio of matrix material to additive material with reflected or emitted electromagnetic radiation from the sample; and output a determined ratio of matrix material to additive material for the measurement.

The ratio of the matrix material to the additive material in the sample may comprise a ratio of the matrix material to one or more of a pigment, filler, or a dye.

The measurement of the electromagnetic radiation reflected or emitted from the sample may comprise light one or more of x-ray fluorescence spectroscopy data, infrared spectroscopy data, near-infrared spectroscopy data, Raman spectroscopy data, terahertz spectroscopy data, x-ray photoelectron spectroscopy data, or energy-dispersive x-ray spectroscopy data.

"And/or" as used herein is defined as the inclusive or v, as specified by the following truth table:

| A | B | AvB |
|---|---|---|
| True | True | True |
| True | False | True |
| False | True | True |
| False | False | False |

The terminology "one or more of A or B" as used herein comprises A, B, or a combination of A and B. The terminology "one or more of A, B, or C" is equivalent to A, B, and/or C. As such, "one or more of A, B, or C" as used herein comprises A individually, B individually, C individually, a combination of A and B, a combination of A and C, a combination of B and C, or a combination of A, B and C.

While the present invention is solely limited by the appended claims, it will be understood that the configurations and/or approaches described herein are exemplary in nature, and that these specific examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

## Claims

1. A method (400) for determining a composition of a coating material on a sample (304), the method (400) comprising:
obtaining calibration data that correlates a ratio of matrix material to additive material with results of nondestructive spectral analysis;
performing (414) nondestructive spectral analysis on the sample; and
comparing (422) results of the nondestructive spectral analysis on the sample to the calibration data to determine a ratio of matrix material to additive material in the sample,
wherein:
obtaining the calibration data that correlates the ratio of matrix material to additive material with results of nondestructive spectral analysis comprises:
creating (402) a plurality of reference standards by applying a reference material to a reference substrate, wherein the reference material comprises a different ratio of a matrix material to an additive material for each of the plurality of reference standards;
performing (406) nondestructive spectral analysis on each of the plurality of reference standards; and
subjecting (408) each of the plurality of reference standards to destructive analysis to characterize the ratio of the matrix material to the additive material in each of the plurality of reference standards, and to correlate results of the destructive analysis and the nondestructive spectral analysis,
subjecting each of the plurality of reference standards to the destructive analysis comprises performing (410) thermogravimetric analysis, and
the coating material is a paint and/or primer.

2. The method of claim 1, wherein comparing the results of the nondestructive spectral analysis on the sample to the calibration data to determine the ratio of matrix material to additive material in the sample comprises:
comparing the results of the nondestructive spectral analysis on the sample to the correlated results of the destructive analysis and the nondestructive spectral analysis on the plurality of the reference standards to determine the ratio of the matrix material to the additive material in the sample.

3. The method of any preceding claim, wherein subjecting each of the plurality of reference standards to the destructive analysis further comprises performing solution-phase chromatography.

4. The method of any preceding claim, wherein subjecting each of the plurality of reference standards to the destructive analysis further comprises performing NMR spectroscopy.

5. The method of any preceding claim, wherein the number of reference standards is in a range of 2-100.

6. The method of any preceding claim, wherein applying the reference material to the reference substrate comprises applying (404) an amount of the additive material in a range of 0-70 wt.% to the reference substrate.

7. The method of any preceding claim, wherein the composition and number of the plurality of reference standards is determined based upon an expected ratio of the additive material to the matrix material in an experimental sample.

8. The method of any preceding claim, wherein performing the nondestructive spectral analysis on the sample comprises performing (418) the nondestructive spectral analysis on cured paint or cured primer.

9. The method of any preceding claim, wherein performing the nondestructive spectral analysis on the sample comprises performing (420) the nondestructive spectral analysis on a component of an aircraft.

10. The method of any preceding claim, wherein determining the ratio of the matrix material to the additive material in the sample comprises determining (424) a ratio of polymeric resin to the additive material.

11. The method of any preceding claim, wherein determining the ratio of the matrix material to the additive material in the sample comprises determining (428) a ratio of the matrix material to one or more of a pigment, filler, or a dye.

12. The method of any preceding claim, wherein determining the ratio of the matrix material to the additive material in the sample comprises determining (426) a ratio of one or more of an epoxy resin, a polyurethane resin, or an acrylic resin to the additive material.

13. The method of any preceding claim, wherein performing the nondestructive spectral analysis (414) comprises performing (416) one or more of x-ray fluorescence spectroscopy, infrared spectroscopy, near-infrared spectroscopy, Raman spectroscopy, terahertz spectroscopy, x-ray photoelectron spectroscopy, or energy-dispersive x-ray spectroscopy.

14. The method of claim 13, wherein performing the nondestructive spectral analysis (414) comprises performing (416) one or more of x-ray fluorescence spectroscopy, x-ray photoelectron spectroscopy, or energy-dispersive x-ray spectroscopy.

15. The method of any preceding claim, further comprising correlating (412) the ratio of the matrix material to the additive material in the sample to one or more of durability, adhesion, corrosion resistance, color retention, gloss retention, chemical resistance, ease of application, flexibility, elasticity, or abrasion resistance.

## Patentansprüche

1. Verfahren (400) zum Bestimmen einer Zusammensetzung eines Beschichtungsmaterials auf einer Probe (304), wobei das Verfahren (400) Folgendes umfasst:
Erhalten von Kalibrierungsdaten, die ein Verhältnis von Matrixmaterial zu Additivmaterial mit Ergebnissen der zerstörungsfreien Spektralanalyse korrelieren;
Durchführen (414) einer zerstörungsfreien Spektralanalyse an der Probe; und
Vergleichen (422) von Ergebnissen der zerstörungsfreien Spektralanalyse an der Probe mit den Kalibrierungsdaten, um ein Verhältnis von Matrixmaterial zu Additivmaterial in der Probe zu bestimmen,
wobei:
das Erhalten der Kalibrierungsdaten, die das Verhältnis von Matrixmaterial zu Additivmaterial mit Ergebnissen der zerstörungsfreien Spektralanalyse korrelieren, Folgendes umfasst:
Herstellen (402) einer Vielzahl von Referenzstandards durch Aufbringen eines Referenzmaterials auf ein Referenzsubstrat, wobei das Referenzmaterial für jeden der Vielzahl von Referenzstandards ein anderes Verhältnis eines Matrixmaterials zu einem Additivmaterial aufweist;
Durchführen (406) einer zerstörungsfreien Spektralanalyse an jedem der Vielzahl von Referenzstandards; und
Unterziehen (408) jedes der Vielzahl von Referenzstandards einer zerstörenden Analyse, um das Verhältnis des Matrixmaterials zu dem Additivmaterial in jedem der Vielzahl von Referenzstandards zu charakterisieren und um Ergebnisse der zerstörenden Analyse mit denen der zerstörungsfreien Spektralanalyse zu korrelieren,
wobei das Unterziehen jedes der Vielzahl von Referenzstandards der zerstörenden Analyse das Durchführen (410) einer thermogravimetrischen Analyse umfasst, und
das Beschichtungsmaterial eine Farbe und/oder Grundierung ist.

2. Verfahren nach Anspruch 1, wobei das Vergleichen der Ergebnisse der zerstörungsfreien Spektralanalyse an der Probe mit den Kalibrierungsdaten, um das Verhältnis von Matrixmaterial zu Additivmaterial in der Probe zu bestimmen, Folgendes umfasst:
Vergleichen der Ergebnisse der zerstörungsfreien Spektralanalyse an der Probe mit den korrelierten Ergebnissen der zerstörenden Analyse und der zerstörungsfreien Spektralanalyse an der Vielzahl der Referenzstandards, um das Verhältnis des Matrixmaterials zu dem Additivmaterial in der Probe zu bestimmen.

3. Verfahren nach einem vorstehenden Anspruch, wobei das Unterziehen jedes der Vielzahl von Referenzstandards einer zerstörenden Analyse weiter das Durchführen einer Lösungsphasenchromatographie umfasst.

4. Verfahren nach einem vorstehenden Anspruch, wobei das Unterziehen jedes der Vielzahl von Referenzstandards einer zerstörenden Analyse weiter das Durchführen einer NMR-Spektroskopie umfasst.

5. Verfahren nach einem vorstehenden Anspruch, wobei die Anzahl der Referenzstandards im Bereich von 2 bis 100 liegt.

6. Verfahren nach einem vorstehenden Anspruch, wobei das Aufbringen des Referenzmaterials auf das Referenzsubstrat das Aufbringen (404) einer Menge des Additivmaterials im Bereich von 0-70 Gew.-% auf das Referenzsubstrat umfasst.

7. Verfahren nach einem vorstehenden Anspruch, wobei die Zusammensetzung und die Anzahl der Vielzahl von Referenzstandards auf der Grundlage eines erwarteten Verhältnisses des Additivmaterials zu dem Matrixmaterial in einer Versuchsprobe bestimmt werden.

8. Verfahren nach einem vorstehenden Anspruch, wobei das Durchführen der zerstörungsfreien Spektralanalyse an der Probe das Durchführen (418) der zerstörungsfreien Spektralanalyse an ausgehärteter Farbe oder ausgehärteter Grundierung umfasst.

9. Verfahren nach einem vorstehenden Anspruch, wobei das Durchführen der zerstörungsfreien Spektralanalyse an der Probe das Durchführen (420) der zerstörungsfreien Spektralanalyse an einem Bauteil eines Luftfahrzeugs umfasst.

10. Verfahren nach einem vorstehenden Anspruch, wobei das Bestimmen des Verhältnisses des Matrixmaterials zu dem Additivmaterial in der Probe das Bestimmen (424) eines Verhältnisses von Polymerharz zu dem Additivmaterial umfasst.

11. Verfahren nach einem vorstehenden Anspruch, wobei das Bestimmen des Verhältnisses des Matrixmaterials zu dem Additivmaterial in der Probe das Bestimmen (428) eines Verhältnisses des Matrixmaterial zu einem oder mehreren von einem Pigment, einem Füllstoff oder einem Farbstoff umfasst.

12. Verfahren nach einem vorstehenden Anspruch, wobei das Bestimmen des Verhältnisses des Matrixmaterials zu dem Additivmaterial in der Probe das Bestimmen (426) eines Verhältnisses von einem oder mehreren von einem Epoxidharz, einem Polyurethanharz oder einem Acrylharz zu dem Additivmaterial umfasst.

13. Verfahren nach einem vorstehenden Anspruch, wobei das Durchführen der zerstörungsfreien Spektralanalyse (414) das Durchführen (416) von einer oder mehreren von einer Röntgenfluoreszenzspektroskopie, Infrarotspektroskopie, Nahinfrarotspektroskopie, Raman-Spektroskopie, Terahertz-Spektroskopie, Röntgenphotoelektronenspektroskopie oder energiedispersiven Röntgenspektroskopie.

14. Verfahren nach Anspruch 13, wobei das Durchführen der zerstörungsfreien Spektralanalyse (414) das Durchführen (416) von einer oder mehreren von einer Röntgenfluoreszenzspektroskopie, Röntgenphotoelektronenspektroskopie oder energiedispersiven Röntgenspektroskopie.

15. Verfahren nach einem vorstehenden Anspruch, weiter umfassend das Korrelieren (412) des Verhältnisses des Matrixmaterials zu dem Additivmaterial in der Probe mit einem oder mehreren von Haltbarkeit, Haftung, Korrosionsbeständigkeit, Farberhalt, Glanzerhalt, Chemikalienbeständigkeit, Anwendungsfreundlichkeit, Flexibilität, Elastizität oder Abriebfestigkeit.

## Revendications

1. Procédé (400) de détermination d'une composition d'un matériau de revêtement sur un échantillon (304), le procédé (400) comprenant les étapes consistant à :
obtenir des données d'étalonnage qui mettent en corrélation un rapport entre le matériau de matrice et le matériau additif et des résultats d'une analyse spectrale non destructive ;
effectuer (414) une analyse spectrale non destructive sur l'échantillon ; et
comparer (422) des résultats de l'analyse spectrale non destructive de l'échantillon aux données d'étalonnage pour déterminer un rapport entre le matériau de matrice et le matériau additif dans l'échantillon,
dans lequel :
l'obtention des données d'étalonnage, qui mettent en corrélation le rapport entre le matériau de matrice et le matériau additif et des résultats de l'analyse spectrale non destructive, comprend les étapes consistant à :
créer (402) une pluralité d'étalon de référence en appliquant un matériau de référence à un substrat de référence, dans lequel le matériau de référence comprend un rapport différent d'un matériau de matrice à un matériau additif pour chacun de la pluralité d'étalons de référence ;
effectuer (406) une analyse spectrale non destructive sur chacun de la pluralité d'étalons de référence ; et
soumettre (408) chacun de la pluralité d'étalons de référence à une analyse destructive afin de caractériser le rapport entre le matériau de matrice et le matériau additif dans chacun de la pluralité d'étalons de référence, et de mettre en corrélation des résultats de l'analyse destructive et de l'analyse spectrale non destructive,
soumettre chacun de la pluralité d'étalons de référence à l'analyse destructive comprend une réalisation (410) d'une analyse thermogravimétrique, et
le matériau de revêtement est une peinture et/ou un apprêt.

2. Procédé selon la revendication 1, dans lequel la comparaison des résultats de l'analyse spectrale non destructive sur l'échantillon aux données d'étalonnage pour déterminer le rapport entre le matériau de matrice et le matériau additif dans l'échantillon comprend l'étape consistant à :
comparer les résultats de l'analyse spectrale non destructive sur l'échantillon aux résultats corrélés de l'analyse destructive et de l'analyse spectrale non destructive sur la pluralité des étalons de référence afin de déterminer le rapport du matériau de matrice au matériau additif dans l'échantillon.

3. Procédé selon une quelconque revendication précédente, dans lequel la soumission de chacun de la pluralité d'étalons de référence à l'analyse destructive comprend en outre une réalisation d'une chromatographie en phase solution.

4. Procédé selon une quelconque revendication précédente, dans lequel la soumission de chacun de la pluralité d'étalons de référence à l'analyse destructive comprend en outre une réalisation d'une spectroscopie RMN.

5. Procédé selon une quelconque revendication précédente, dans lequel le nombre d'étalons de référence est dans une plage de 2 à 100.

6. Procédé selon une quelconque revendication précédente, dans lequel l'application du matériau de référence au substrat de référence comprend une application (404) d'une quantité du matériau additif dans une plage de 0 à 70 % en poids au substrat de référence.

7. Procédé selon une quelconque revendication précédente, dans lequel la composition et le nombre de la pluralité d'étalons de référence sont déterminés en fonction d'un rapport attendu entre le matériau additif et le matériau de matrice dans un échantillon expérimental.

8. Procédé selon une quelconque revendication précédente, dans lequel l'exécution de l'analyse spectrale non destructive sur l'échantillon comprend une exécution (418) de l'analyse spectrale non destructive sur de la peinture durcie ou de l'apprêt durci.

9. Procédé selon une quelconque revendication précédente, dans lequel l'exécution de l'analyse spectrale non destructive sur l'échantillon comprend une exécution (420) de l'analyse spectrale non destructive sur un composant d'un aéronef.

10. Procédé selon une quelconque revendication précédente, dans lequel la détermination du rapport du matériau de matrice au matériau additif dans l'échantillon comprend une détermination (424) d'un rapport de résine polymère au matériau additif.

11. Procédé selon une quelconque revendication précédente, dans lequel la détermination du rapport du matériau de matrice au matériau additif dans l'échantillon comprend une détermination (428) d'un rapport du matériau de matrice à un ou plusieurs parmi un pigment, une charge ou un colorant.

12. Procédé selon une quelconque revendication précédente, dans lequel la détermination du rapport du matériau de matrice au matériau additif dans l'échantillon comprend une détermination (426) d'un rapport d'une ou plusieurs parmi une résine époxy, une résine polyuréthane ou une résine acrylique au matériau additif.

13. Procédé selon une quelconque revendication précédente, dans lequel l'exécution de l'analyse spectrale non destructive (414) comprend une exécution (416) d'une ou plusieurs parmi une spectroscopie de fluorescence X, une spectroscopie infrarouge, une spectroscopie proche infrarouge, une spectroscopie Raman, une spectroscopie térahertz, une spectroscopie photoélectronique X ou une spectroscopie X à dispersion d'énergie.

14. Procédé selon la revendication 13, dans lequel l'exécution de l'analyse spectrale non destructive (414) comprend une exécution (416) d'une ou plusieurs parmi une spectroscopie de fluorescence X, une spectroscopie photoélectronique X ou une spectroscopie X à dispersion d'énergie.

15. Procédé selon une quelconque revendication précédente, comprenant en outre une mise en corrélation (412) du rapport du matériau de matrice au matériau additif dans l'échantillon avec une ou plusieurs parmi la durabilité, l'adhérence, la résistance à la corrosion, la rétention de couleur, la rétention de brillant, la résistance chimique, la facilité d'application, la flexibilité, l'élasticité ou la résistance à l'abrasion.
